Europäisches Patentamt

European Patent Office

Office européen des brevets

(19)

(11) Publication number: **0 181 966**
**A1**

## (12) EUROPEAN PATENT APPLICATION

(21) Application number: **84201638.8**

(22) Date of filing: **13.11.84**

(51) Int. Cl.⁴: **A 61 K 9/28**

(43) Date of publication of application: **28.05.86**
**Bulletin 86/22**

(84) Designated Contracting States: **NL**

(71) Applicant: **Gist - Brocades N.V., Wateringseweg 1 P.O. Box 1, NL-2600 MA Delft (NL)**

(72) Inventor: **Groenendaal, Jan Willem, Sandenburg 170, NL-2036 PV Haarlem (NL)**
Inventor: **Sijbrands, Gerrit Jan, Piet Leffertstraat 8, NL-2042 EH Zandvoort (NL)**

(74) Representative: **Schmieman, Johannes Hendrik et al, Gist-Brocades N.V. Patents & Trademarks Department Martinus Nijhofflaan 2 P.O. Box 1, NL-2600 MA Delft (NL)**

(54) **Compression-coated dispersible tablets.**

(57) Tablets which yield a fine dispersion when immersed in water, consisting of a quickly dispersible core containing the active substance or substances, covered by a compression coating which is also quickly dispersible.

EP 0 181 966 A1

0181966

Gist-brocades

Compression-coated dispersible tablets.

This invention relates to tablets which yield a fine dispersion when immersed in water, such as medicinal tablets, intended to be dispersed in water to form a drinkable mixture.

The invention also relates to a process of producing the dispersible tablets.

There are many existing and thinkable possibilities for conveying oral medication, each suitable to certain specific requirements. Besides the solid dosage forms such as tablets and capsules, there is also an increasing demand for liquid, drinkable preparations. This is especially the case with the elderly and with children, and the more so when large doses of the medicament are to be taken frequently and/or for extended periods.

Drinkable preparations can sometimes be delivered to the patient already in their liquid form, in solution, suspension or emulsion. This always has obvious logistic disadvantages, and moreover many medicaments tend then to decompose quite quickly.

A good alternative is to supply the medicament in a special solid formulation, which on putting into water will quickly disintegrate to form a mixture which is homogeneous and agreeable enough to be drinkable. Examples are effervescent tablets and dispersion tablets, the former depending on the reaction of bicarbonate or carbonate with an acid or on other excipients having the capacity of developing gas after contact with water, the latter on the presence of disintegrating agents having the capacity to swell with water. While many medicaments are incompatible with bicarbonate and/or with acids, the dispersion tablets are the more generally suitable of the two sorts. One example of the latter is described in U.K. Patent 2,067,900, which is directed to a dispersible tablet containing as medicaments trimethoprim and sulphamethoxazole, and as disintegrating agent cross-linked polyvinylpyrrolidone.

Another problem occurring independently from that of making a drinkable formulation, is the need to protect certain active substances against loss of substance, of potency and/or physico-chemical characteristics due to contact with the atmosphere, recrystallization and/or sublimation. For this purpose, several devices have been constructed, such as sugar-coated, film-coated and compression-coated tablets, capsules and micro-capsules. The latter are quite expensive to produce and also tend to decreased bioavailability, while the other forms of protection hitherto devised, almost by definition, were to be considered incompatible with the principle of the dispersible tablet, i.e. the ability to rapidly disintegrate in water. Therefore, any coating of dispersible tablets has not been described before.

It has now surprisingly been found possible to combine the desired properties of rapid disintegration in water with protection against the detrimental influences described in the previous paragraph.

According to the invention this is achieved by covering a quickly dispersible core with a compression coating which is also quickly dispersible. The coating may suitably be made quickly dispersible by incorporating in it one or more disintegrating agents. Examples of suitable disintegrating agents to be used in the coating are:

a. cross-linked polyvinylpyrrolidones (such as POLYPLASDONE-XL®, KOLLIDON-CL®);

b. cross-linked sodium carboxymethyl celluloses (such as Ac-Di-Sol®);

c. sodium starch glycollates (such as PRIMOJEL®, EXPLOTAB®);

d. ion-exchange resins such as AMBERLITE-IRP-88®;

e. microcrystalline celluloses (such as AVICEL®

PH-1O1, PH-1O2);

      f. compressible starches (such as STARCH-1500®);

      g. starch and modified starches;

      h. alginic acid and derivatives;

      i. formaldehyde-casein (such as ESMA-SPRENG®).

In particular, cross-linked polyvinylpyrrolidone, cross-linked sodium carboxymethylcellulose and sodium starch glycollate have been found very useful.

The composition of the core may be similar to the composition of dispersible tablets generally known in pharmaceutical practice. Beside the active substance and the disintegrating agent it may contain other substances commonly used in pharmacy, such as binding agents (e.g. methylcellulose, polyvinyl pyrrolidone), granulation aids (e.g. magnesium carbonate), gliding agents (e.g. silicium dioxide preparations), lubricants (e.g. magnesium stearate), fillers (e.g. lactose and microcrystalline cellulose), taste improvers and dyes.

The active substance(s) preferably constitute(s) a maximum of 70 percent by weight of the core, more preferably not more than 65%.

The compression coating of the tablets may, beside the disintegrating agents, contain other pharmaceutical excipients, such as binding agents, gliding agents, lubricants, fillers, taste improvers and dyes.

It will be appreciated that the dispersible coating, while protecting the core, is in turn sensitive to atmospheric humidity, against which it can be protected by methods known in the art, such as blister packs.

The tablets of the invention are particularly suitable for active substances which are prone to sublimation and/or recrystallisation.

One particular example of such an active substance is cyclandelate.

Cyclandelate, a well-known vasoactive substance, finds its medical application primarily with elderly patients. During the many years this drug has been used, its field of application has gradually broadened, the recommended daily dosage increased and so did the duration of treatment. Currently, patients are taking cyclandelate in daily doses of up to 1600 mg, for years on end. As cyclandelate tends to sublimate and to recristallize on contact with the atmosphere, its existing formulations are sugar-coated tablets and capsules. When the demand was expressed for a drinkable formulation, the dispersible tablets according to the invention proved very suitable.

Tablets containing cyclandelate are therefore a preferred feature of the invention.

The cyclandelate containing tablets, prepared according to the invention, have a preferable content of 400-1200, more preferably of 800 mg of cyclandelate.

The invention includes a process for the preparation of the tablets. The process comprises the steps commonly used in the preparation of compression-coated tablets, and is characterised by the preparation of a quickly dispersible core, containing the medicament, and covering said core with a compression coating, which is also quickly dispersible.

The idea of compression-coating itself is quite old, going back to P.J. Noyes' British Patent 859,996 (1896). In 1937 F. Kilian, a German inventor, received British Patent 464,903 for a combination of two machines running synchronously to produce compression-coated tablets. The principle of this patent has been adopted by the currently available Manesty Dry Cota® apparatus, while Kilian himself developed a different idea of a single rotary press designed to perform only the coating step which is currently on sale as the Prescoter®. This machine was used in the process of the invention, but because it (as any other available machine designed for compression-coating) was only capable of handling much smaller cores than the required dispersion tablets containing 800 mg of cyclandelate (which cores have a diameter of about 14 mm and a thickness of more than 8 mm) a special adaptation had to be made at the Prescoter®.

In the Kilian Prescoter® the cores are fed to a rotating core-collecting disc from a vibrating hopper through a glass tube. In the adapted apparatus the cores are first guided from the hopper to a core-delivering disc, provided with cavities to receive the cores, which is driven by a stepping motor running synchronously with the press. The hopper is connected with the disc by a slant open slide, consisting of a number of thin metal (preferably stainless steel) rods.

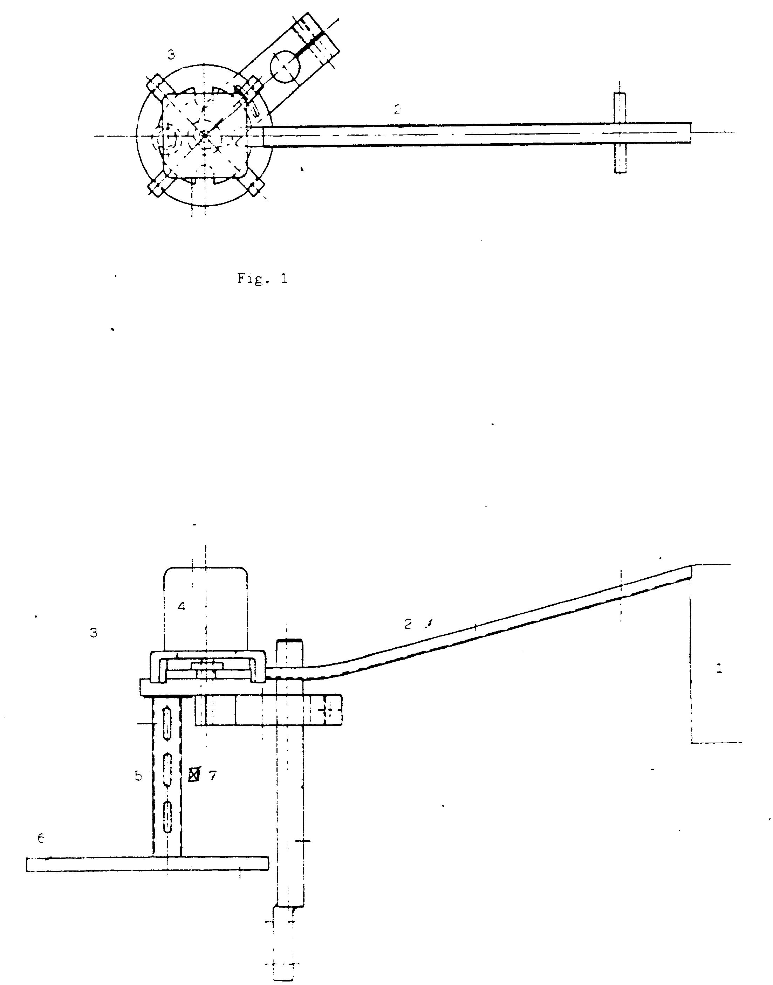

From the core-delivering disc the cores fall on the core-collecting disc through a vertical tube. The construction is illustrated by figures 1/2 and 2/2, respectively a top view and a side view of the adapted part of the apparatus.

In the figures (1) is the vibrating hopper, (2) is the open slide, (3) the core-delivering disc, (4) the stepping motor, (5) the vertical tube and (6) the core-collecting disc. Also a fotocell (7) is installed, connected with appropriate control equipment which will stop the tabletting machine whenever no cores are delivered.

The tablets, coated with this adapted Prescoter® machine, have a total outer diameter of about 18 mm and a thickness of about 9 mm. Their compressed coating is about 1.5 mm thick.

The following Examples are intended to illustrate the invention, without in any way being exhaustive.

## Example 1

A dispersible tablet core was prepared as follows:

50 kg of cyclandelate having a particle size smaller than 0.71 mm (25 mesh) were mixed with 1.25 kg of magnesium carbonate and 9 kg of aqueous 1.2% methyl cellulose. The mass was granulated and dried, and subsequently passed through an oscillating sieve of 0.71 mm (25 mesh). The granules were then mixed with:

| | |
|---|---|
| PRIMOJEL® (sodium starch glycollate) | 2.5 kg |
| KOLLIDON CL® (crosslinked polyvinylpyrrolidone) | 5.38 kg |
| AVICEL® PH-102 (microcrystalline cellulose) | 11.6 kg |
| LEVILITE® (silicium dioxide) | 1.9 kg |
| AEROSIL 200 V® (silicium dioxide) | 1.05 kg |
| Magnesium stearate | 0.38 kg |
| Saccharin sodium | 0.94 kg |
| CAPSAROMA® (natural peppermint flavour mc-92505) | 0.94 kg |

This mixture was then pressed into dispersible tablet cores, flat with facetted rims, having a diameter of 14 mm, a thickness of 8.3 mm, weighing 1215 mg and containing 800 mg of cyclandelate. The hardness, tested in a Schleuniger type 2-E hardness-tester, was 60 newton. On putting into 30 ml of water at 20°C, these cores disintegrated completely in 15 to 25 seconds.

Example 2

A mixture for a dispersible compression-coating was prepared by mixing together (by weight):

| | |
|---|---|
| KOLLIDON CL® (crosslinked polyvinylpyrrolidone) | 19.5 % |
| AVICEL® PH-102 (microcrystalline cellulose) | 69.0 % |
| AEROSIL 200 V® (silicium dioxide) | 0.5 % |
| Magnesium stearate | 1.0 % |
| Lactose DC 10 (direct compressible lactose) | 10.0 % |

This mixture was fed, together with the cores prepared according to Example 1, to the Kilian Prescoter®, which was especially adapted as described before. The ensuing dispersible compression-coated tablets had an outer diameter of 18 mm, a thickness of 9 mm, the coating being 1.5 mm thick and the total weight about 2.28 gram. On putting into 30 ml of water of 20°C, these tablets disintegrated completely in less than 90 seconds, producing after simple stirring a homogeneous, drinkable suspension. The friability, tested in a Roche friabilator (running 40 r.p.m. during 4 minutes), was less than 1%.

## Example 3

A mixture for a dispersible compression-coating was prepared by mixing together (by weight):

| | |
|---|---|
| KOLLIDON CL® | 19.5 % |
| AVICEL® PH-102 | 64.0 % |
| AEROSIL 200 V® | 0.5 % |
| Magnesium stearate | 1.0 % |
| Lactose DC 10 | 10.0 % |
| Polyethylene glycol 6000 | 5.0 % |

This mixture was fed, together with the cores prepared according to Example 1, to the Kilian Prescoter ® which was especially adapted as described before. The ensuing dispersible compression-coated tablets had the same properties as specified under Example 2.

## Example 4

A mixture for a dispersible compression-coating was prepared by mixing together (by weight):

| | |
|---|---|
| KOLLIDON CL® | 19.5 % |
| AVICEL® PH-102 | 64.0 % |
| AEROSIL 200 V® | 0.5 % |
| Magnesium stearate | 1.0 % |
| Lactose DC 10 | 10.0 % |
| LHPC LH 11 (low substituted hydroxy propyl cellulose) | 5.0 % |

This mixture was fed, together with the cores prepared according to Example 1, to the Kilian Prescoter ® which was especially adapted as described before. The ensuing dispersible compression-coated tablets had the same properties as specified under Example 2.

## Example 5

A mixture for a dispersible compression-coating was prepared by mixing together (by weight):

| | |
|---|---|
| KOLLIDON CL® | 19.5 % |
| AVICEL® PH-102 | 50.0 % |
| AEROSIL 200 V® | 0.5 % |
| Magnesium stearate | 1.0 % |
| Lactose DC 10 | 29.0 % |

This mixture was fed, together with the cores prepared according to Example 1, to the Kilian Prescoter®, which was especially adapted as described before. The ensuing dispersible compression-coated tablets had the same properties as specified under Example 2.

Claims

1. Tablets which yield a fine dispersion when
immersed in water, characterised in that a quickly dispersible
core containing the active substance or substances, is covered
by a compression coating which is also quickly dispersible.

2. Compression-coated tablets according to claim 1,
characterised in that the compression-coating contains one or
more disintegrating agents.

3. Compression-coated tablets according to claim 2,
characterised in that the disintegrating agent or agents in
the coating are chosen from the group consisting of

      a. cross-linked polyvinylpyrrolidone

      b. cross-linked sodium carboxymethylcelluloses

      c. sodium starch glycollates

      d. ion-exchange resins

      e. microcrystalline celluloses

      f. compressible starches

      g. starch and modified starches

      h. alginic acid and derivatives.

      i. formaldehyde-caseins.

4. Compression-coated tablets according to claim 3,
characterised in that the disintegrating agent is cross-linked
polyvinylpyrrolidone.

5. Compression-coated tablets according to claim 3,
characterised in that the disintegrating agent is cross-linked
carboxymethylcellulose.

6. Compression-coated tablets according to claim 3,
characterised in that the disintegrating agent is sodium
starch glycollate.

7. Compression-coated tablets according to any of
the foregoing claims, characterised in that the active
substance or substances are present in the core in a
concentration not exceeding 70% by weight.

8. Compression-coated tablets according to any of
the foregoing claims, characterised in that the active
substance or substances, when unprotected, are prone to
sublimation and/or recrystallisation.

9. Compression-coated tablets according to claim 8, characterised in that the active substance in the core is cyclandelate.

10. Compression-coated tablets according to claim 9, characterised in that cyclandelate is present in a dosage of 400 to 1200 mg per tablet.

11. Compression-coated tablets according to claim 10, characterised in that cyclandelate is present in the core in a dosage of 800 mg per tablet.

12. Compression-coated tablets according to any of the foregoing claims, characterised in that their outer diameter is about 18 mm, their thickness about 9 mm, and their compressed coating is about 1.5 mm thick.

13. Process for the preparation of the compression-coated medicinal tablets as described in any of the foregoing claims, characterised by the preparation of a quickly dispersible core, containing the medicament, and covering said core with a compression-coating, which is also quickly dispersible.

0181966

i/i

Fig. 1

Fig. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-1 811 809 (SCHEPKY et al.) <br><br> * Page 1, line 5 - page 2; page 3, example 1; page 6, example 8; claims * | 1-3,7, 12,13 | A 61 K 9/28 |
| Y | | 4-6,8-11 | |
| X | US-A-3 019 169 (KLUMPP et al.) <br><br> * Column 1, lines 42-53; column 2, lines 3-48; columns 5,6, example 4; claims * | 1-3,7, 12,13 | |
| Y | | 4-6,8-11 | |
| | | | TECHNICAL FIELDS SEARCHED (Int. Cl.4) |
| Y | EP-A-0 003 589 (THE WELLCOME FOUNDATION LTD.) <br> * Page 2, lines 16-21; page 6, line 18 - page 7, line 22; page 10, line 2 - page 11, line 18; claims 10,11 * | 3-6 | A 61 K |
| Y | NL-C- 96 376 (N.V. KON. PHARM. FABRIEKEN) <br> * Column 3; lines 1-6; claims * | 8-11 | |
| | --- -/- | | |

The present search report has been drawn up for all claims

| Place of search | Date of completion of the search | Examiner |
|---|---|---|
| THE HAGUE | 25-07-1985 | BENZ K.F. |

## EUROPEAN SEARCH REPORT

**European Patent Office**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-3 033 754 (KRAHNKE et al.) * Column 1, line 26 - column 2, line 43; claims * | 8 | |
| | ----- | | |

**TECHNICAL FIELDS SEARCHED (Int. Cl.4)**

The present search report has been drawn up for all claims

| Place of search | Date of completion of the search | Examiner |
|---|---|---|
| THE HAGUE | 25-07-1985 | BENZ K.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO Form 1503 03.82